# FASCICULE DE BREVET EUROPEEN

(11) **EP 3 658 115 B1**
(45) Date de publication et mention de la délivrance du brevet: **23.02.2022**
(21) Numéro de dépôt: 18746942.4
(22) Date de dépôt: 27.07.2018
(51) Int. Cl.: A61K 38/26, A61K 38/28, A61K 45/06, A61K 47/02, A61K 9/08, A61K 31/198, C07K 14/62, A61P 3/10, A61P 5/48

(54) **COMPOSITIONS SOUS FORME D'UNE SOLUTION AQUEUSE INJECTABLE COMPRENANT AU MOINS L'INSULINE HUMAINE A21G ET UN SUPPRESSEUR DE GLUCAGON A ACTION PRANDIALE**
ZUSAMMENSETZUNGEN IN FORM EINER INJIZIERBAREN WÄSSRIGEN LÖSUNG MIT MINDESTENS EINEM HUMANINSULIN A21G UND EINEM SUPPRESSOR VON POSTPRANDIALEM GLUCAGON
COMPOSITIONS IN THE FORM OF AN INJECTABLE AQUEOUS SOLUTION COMPRISING AT LEAST HUMAN INSULIN A21G AND A PRANDIAL ACTION GLUCAGON SUPPRESSOR

(30) Priorité: 27.07.2017 FR 1757172; 28.07.2017 FR 1757184; 14.06.2018 FR 1855250
(43) Date de publication de la demande: 03.06.2020
(73) Titulaire: Adocia, 69003 Lyon (FR)
(72) Inventeur: CHAN, You-Ping, 69360 Ternay (FR)
(74) Mandataire: Tripoz, Inès
(86) Numéro de dépôt international: PCT/EP2018/070510
(87) Numéro de publication internationale: WO 2019/020820

(56) Documents cités:
- WO-A1-2007/104786
- WO-A1-2007/135118
- US-A1- 2009 192 075

## Description

L'invention concerne les thérapies par injection de composition comprenant au moins de l'insuline humaine A21G, à action prandiale, et un suppresseur de glucagon, en particulier à action prandiale, pour traiter le diabète et permettre d'améliorer le contrôle des hyperglycémies post-prandiales.

Le diabète de type 1 est une maladie autoimmune conduisant à la destruction des cellules béta du pancréas. Ces cellules sont connues pour produire l'insuline, dont le rôle principal est de réguler l'utilisation du glucose dans les tissus périphériques (Gerich 1993 Control of glycaemia). Par conséquent, les patients atteints de diabète de type 1 souffrent d'hyperglycémie chronique et doivent s'administrer de l'insuline exogène afin de limiter cette hyperglycémie. L'insulinothérapie a permis de changer drastiquement l'espérance de vie de ces patients.

A ce jour, les patients diabétiques de type 1 utilisent deux types d'insuline, les insulines prandiales à action courte pour contrôler leur glycémie au moment du repas et les insulines basales à action longue pour contrôler leur glycémie tout au long de la journée et de la nuit. Plusieurs types d'insuline à action courte existent caractérisées par leur délai d'action. Par exemple, l'insuline humaine, dite regular, a une action retardée par rapport aux insulines analogues, dites rapides, telles que l'insuline lispro (Humalog^{®}, ELI LILLY) ou l'insuline aspart (Novorapid^{®}, NOVO NORDISK). Ainsi, l'insuline humaine doit être administrée en moyenne 30 minutes avant le repas alors que les insulines analogues peuvent être administrées 15 minutes avant le repas ou au moment du repas. De plus, il est admis que les insulines analogues conduisent à un meilleur contrôle de la glycémie post-prandiale que l'insuline humaine, ce qui explique que la très grande majorité des patients en Europe et aux Etats-Unis utilisent aujourd'hui des insulines analogues rapides dont l'action est plus courte que celle de l'insuline humaine.

Cependant, le contrôle de la glycémie après la prise d'un repas assuré par ces insulines prandiales exogènes n'est pas optimal, même dans le cas des insulines analogues rapides. Ceci est lié, en partie, au fait que ces patients, contrairement aux personnes saines, produisent du glucagon après la prise d'un repas, ce qui conduit au déstockage d'une partie du glucose stocké dans le foie. Cette production de glucose médiée par le glucagon aggrave le problème d'hyperglycémie post-prandiale de ces patients et conduit à une utilisation excessive de l'insuline.

Cette problématique de régulation de la glycémie post-prandiale est assez similaire pour les patients atteints de diabète de type 2 traités par l'insuline dans les cas où leur maladie a conduit à une perte très significative de leur capacité de production d'insuline et d'amyline.

Il a été démontré que des suppresseurs de glucagon, en particulier des peptides et/ou hormones, sont susceptibles d'inhiber la production de glucagon après la prise d'un repas, ce qui conduit à améliorer significativement le contrôle de la glycémie post-prandiale. Par exemple l'amyline, une hormone produite par les cellules béta du pancréas, et dont la production est également déficiente chez les patients diabétiques de type 1, joue un rôle clé dans la régulation de la glycémie post-prandiale. L'amyline, également connue sous le nom de « islet amyloid polypeptide » ou IAPP, est un peptide de 37 amino-acides qui est co-stocké et co-sécrété avec l'insuline (Schmitz 2004 Amylin Agonists). Ce peptide est décrit comme bloquant la production du glucagon par les cellules alpha du pancréas. Ainsi, l'insuline et l'amyline ont des rôles complémentaires et synergiques, puisque l'insuline permet de réduire la concentration de glucose dans le sang alors que l'amyline permet de réduire l'entrée de glucose endogène dans le sang en inhibant la production, ou sécrétion, du glucagon endogène.

Cependant, l'amyline humaine a des propriétés qui ne sont pas compatibles avec les exigences pharmaceutiques en termes de solubilité et de stabilité (Goldsbury CS, Cooper GJ, Goldie KN, Muller SA, Saafi EL, Gruijters WT, Misur MP, Engel A, Aebi U, Kistler J : Polymorphic fibrillar assembly of human amylin. J Struct Biol 119:17-27, 1997). L'amyline est connue pour former des fibres amyloides qui conduisent à la formation de plaques qui sont insolubles dans l'eau. Par conséquent, il a été nécessaire de développer un analogue afin de résoudre ces problèmes de solubilité.

La société Amylin a développé un analogue de l'amyline, le pramlintide, pour pallier le manque de stabilité physique de l'amyline humaine. Ce produit commercialisé sous le nom de Symlin^{®} a été approuvé en 2005 par la FDA pour le traitement des diabétiques de type 1 et de type 2, en complément d'une insulinothérapie. Il doit être administré par voie sous cutanée trois fois par jour, dans l'heure précédant le repas afin d'améliorer le contrôle de la glycémie post-prandiale, compte tenu de sa demi-vie relativement courte de quelques heures. Ce peptide est formulé à pH 4,0 et est décrit pour fibriller lorsque le pH de la solution est supérieur à 5,5. Des variantes d'analogues sont décrites dans le brevet US5,686,411.

Les analogues d'amyline ou les agonistes au récepteur d'amyline dites « prandiales » ou « à action courte » reproduisent les effets de l'amyline tout en présentant une demi-vie plus importante. Ces dérivés de l'amyline permettant de contrôler la glycémie au moment du repas peuvent présenter une demi-vie inférieure à 8h. Cette demi-vie est la demi-vie apparente d'élimination après injection sous-cutanée chez l'homme. La demi vie de ces analogues d'amyline ou les agonistes au récepteur d'amyline peut être inférieure à 5 heures, notamment inférieure à 4 heures, voire inférieure à 3 heures.

Cependant, les compositions comprenant de l'amyline ou un analogue de l'amyline, et en particulier le pramlintide peuvent provoquer certains effets indésirables chez les patients. En particulier ces compositions peuvent provoquer des nausées chez des patients.

Le GLP-1, un autre peptide physiologique, est également décrit pour jouer un rôle similaire à celui de l'amyline en termes de suppression de la sécrétion du glucagon à la suite de la prise d'un repas. Le GLP-1 est aussi connu pour son rôle d'insuline sécrétagogue et est donc particulièrement efficace en complément de l'insuline, en particulier chez les patients diabétiques de type 2. Ces actions sont glucodépendantes, ce qui minimise le risque hypoglycémique. Les GLP-1 RA, ne sont approuvés, à ce jour, que pour les patients diabétiques de type 2.

De même, le GLP-1 humain ne peut être utilisé comme traitement thérapeutique du fait d'une demi-vie extrêmement courte. Différents dérivés du GLP-1, agonistes du récepteur du GLP-1, dits GLP-1 RA, ou analogues du GLP-1, reproduisent les effets du GLP-1 tout en présentant une demi-vie plus importante. Ces dérivés du GLP-1 peuvent être répartis en trois groupes selon leur demi-vie respective : ceux à action courte, ou prandiale, pour contrôler la glycémie au moment du repas (demi-vie inférieure à 8h), ceux à action quotidienne pour couvrir les besoins au cours de la journée (demi-vie supérieure à 8h, voire 10h) et ceux à action hebdomadaire pour couvrir les besoins au cours de la semaine (demi-vie supérieure à 48h). Au niveau des GLP-1 RA à action prandiale, les deux peptides approuvés à ce jour sont l'exenatide (Byetta^{®}, ASTRA-ZENECA, deux administrations par jour) et le lixisenatide (Lyxumia^{®}, SANOFI, une administration par jour). Ces deux GLP-1 RA sont formulés à un pH proche de 4 et sont à administrer dans l'heure précédent le repas, comme le pramlintide.

Une des principales difficultés pour les patients insulinodépendants à utiliser ces différents composés à action courte suppresseurs de glucagon est liée au nombre d'injections supplémentaires, pouvant aller de 1 à 3 injections par jour au-delà des 2 à 4 injections d'insuline. Il est donc primordial de pouvoir combiner en solution une insuline prandiale avec un suppresseur de glucagon à action prandiale afin de permettre l'utilisation de ces composés complémentaires à l'insuline sans alourdir le poids du traitement pour les patients. De plus cela permettrait de mimer plus finement la physiologie puisque ces hormones sont toutes les deux sécrétées en réponse à un repas, afin d'améliorer le contrôle de la glycémie post-prandiale, en particulier un meilleur contrôle des hyperglycémies post-prandiales, et donc de mieux traiter le diabète.

Or, les insulines prandiales et ces peptides d'intérêt ne sont pas compatibles en solution aqueuse. En effet, les insulines prandiales ont une stabilité chimique optimale à un pH proche de 7 alors que les dérivés de l'amyline ou du GLP-1 ne sont pas stables physiquement et chimiquement à un pH proche du pH physiologique.

Cette difficulté a conduit à la conception de pompe contenant deux réservoirs permettant de maintenir séparés l'insuline prandiale du dérivé de l'amyline. La demande de brevet US2016/001002 de la société ROCHE décrit une telle pompe afin de rendre possible la co-administration de l'amyline et d'une insuline prandiale avec un seul dispositif médical. Cependant, il serait préférable de pouvoir mélanger en solution aqueuse ces hormones afin d'employer des dispositifs médicaux existants, plus simples et/ou présentant un risque moindre de dysfonctionnement.

Une autre solution apportée au problème du mélange en solution aqueuse de ces hormones consiste à substituer l'eau par un solvant organique. La demande de brevet WO2013067022 de la société XERIS décrit des compositions qui comprennent de l'amyline et de l'insuline en solution dans un solvant organique. Cependant, l'utilisation d'un solvant organique tel que le DMSO soulève des questions de sécurité pour le patient pour le traitement de maladie chronique telle que le diabète. En outre les solvants organiques peuvent être problématiques par rapport aux dispositifs d'injections, notamment en solubilisant certains composants de ceux-ci. Il est donc souhaitable de développer ces combinaisons sous forme de solution aqueuse.

Le problème de stabilité en solution aqueuse a également été contourné en préparant des mélanges à l'état solide. La demande de brevet EP2060268 de NOVO NORDISK décrit des formulations d'insuline et de pramlintide sous forme de poudre atomisée pour l'application nasale. Cependant, la voie d'administration préférable et la plus utilisée à ce jour est la voie sous-cutanée pour laquelle l'obtention de solutions aqueuses prêtes à l'emploi est nécessaire.

Une autre approche a consisté à combiner une insuline prandiale à un dérivé de l'amyline au pH auquel ce dérivé de l'amyline est stable physiquement. La demande de brevet US20090192075 de la société Biodel décrit une composition liquide comprenant l'insuline humaine, le pramlintide et un chélateur de zinc à pH 4,0. Cette demande décrit une action rapide de l'insuline humaine par la présence d'un chélatant de zinc. Cependant, l'insuline humaine étant connue pour ne pas être chimiquement stable à pH acide, cette technique ne devrait pas permettre de satisfaire les critères de la Pharmacopée EP et/ou US.

Une approche alternative consiste à modifier la structure des insulines prandiales pour améliorer leur stabilité à des pH acides. La demande WO2007104786 exemplifie des compositions comprenant les insulines analogues rapides A21G, B28D desB30 et A21G, B28E desB30 qui sont solubles à pH acide.

La demande WO2007104786 présente également des compositions d'insuline rapides, notamment B28D (insuline aspart) à pH neutre et en présence de tensioactif, et en particulier de dérivé du glycérophosphate, plus particulièrement le dimyristoyl glycérophosphoglycérol (DMPG), conduisent à des stabilités mesurées par ThT bien supérieures à celles des compositions d'insulines analogues rapides A21G, B28D desB30 et A21G, B28E desB30 à pH acide.

Les compositions de l'art antérieur comprenant en association une insuline prandiale et le pramlintide décrivent, la plupart du temps les différents types d'insulines prandiales, cependant leurs exemples sont dirigés vers des compositions comprenant des insulines analogues prandiales dites rapides, ces dernières étant considérées comme plus performantes que l'insuline humaine.

De façon surprenante, la demanderesse a démontré qu'une composition contenant l'insuline humaine A21G, dite « regular », c'est-à-dire une insuline différant de l'insuline humaine uniquement par le remplacement du résidu asparagine en position 21 sur la chaine A par un résidu glycine, moins rapide que les analogues d'insuline dits « rapides », en association avec un suppresseur de glucagon à action prandiale à un pH allant de 3,5 à 4,4 en solution aqueuse permettait d'obtenir un meilleur contrôle de la glycémie post-prandiale qu'avec un analogue d'insuline prandiale dit rapide.

WO2007/135118 A1 mentionne l'insuline A21G dans une longue liste d'insulines mutées.

De plus, La demanderesse a démontré, de manière surprenante, que ladite composition contenant l'insuline humaine A21G, en association avec un suppresseur de glucagon à action prandiale à un pH allant de 3,5 à 4,4 en solution aqueuse présentait une stabilité physique et chimique compatible avec les exigences pharmaceutiques et supérieure aux solutions proposées dans l'art antérieur.

L'obtention d'une composition sous forme de solution aqueuse injectable présentant un meilleur contrôle des hyperglycémies post-prandiales et des propriétés de stabilités physique et chimique améliorées par rapport à celles décrites dans l'art antérieur, est remarquable car il est bien connu de l'homme de l'art que dans le cas de combinaisons les pharmacocinétiques des produits en associations et les propriétés de stabilité physique et chimique sont très difficiles à prédire.

Par ailleurs, il est également recherché une composition qui permette de diminuer, voire de supprimer, tout ou partie des effets indésirables qui peuvent être générés par les principes actifs.

L'invention concerne une composition sous forme d'une solution aqueuse injectable, dont le pH est compris de 3,5 à 4,4, comprenant au moins l'insuline humaine A21G dite regular et au moins un suppresseur de glucagon à action prandiale.

Selon un mode de réalisation, le suppresseur de glucagon à action prandiale est un analogue de l'amyline ou un agoniste du récepteur de l'amyline, un analogue du GLP-1 ou un agoniste du récepteur au GLP-1, encore appelé GLP-1 RA.

La demanderesse a observé que la formulation selon l'invention à pH compris de 3,5 à 4,4 possède des propriétés pharmacocinétiques compatibles avec une utilisation au moment des repas et permet un meilleur contrôle de la glycémie postprandiale.

En outre, la demanderesse a mis en évidence que ces formulations conduisaient à un ralentissement de l'absorption du pramlintide. Cela est notamment caractérisé par un pic plasmatique (tmax) de pramlintide significativement retardé et/ou par une exposition plasmatique au pramlintide précoce (AUC₀₋₃₀ₘᵢₙ) significativement diminuée par rapport à l'administration de pramlintide seul.

Ce ralentissement permet de diminuer voire de supprimer les effets indésirables du pramlintide, en particulier en ce qui concerne les nausées.

L'invention concerne également l'utilisation d'une composition sous forme d'une solution aqueuse injectable, dont le pH est compris de 3,5 à 4,4 comprenant au moins l'insuline humaine A21G et un suppresseur de glucagon, en particulier à action prandiale, pour améliorer le contrôle de la glycémie postprandiale.

L'invention concerne une également une composition selon l'invention destinée à être utilisée dans une méthode de traitement du diabète caractérisée en ce qu'elle est administrée en bolus avant les repas.

L'invention concerne une également une composition selon l'invention destinée à être utilisée dans une méthode de traitement du diabète caractérisée en ce qu'elle est administrée pour améliorer le contrôle de la glycémie postprandiale.

L'invention concerne une également une composition selon l'invention destinée à être utilisée dans une méthode de traitement du diabète caractérisée en ce qu'elle est administrée pour améliorer le contrôle de la glycémie postprandiale et pour diminuer les effets indésirables du pramlintide.

L'invention concerne une également une composition selon l'invention destinée à être utilisée dans une méthode de traitement du diabète caractérisée en ce qu'elle permet de diminuer la prise alimentaire induite par l'insuline.

Selon un mode de réalisation, la diminution de la prise alimentaire concerne la période allant de l'injection à 4 heures après l'injection.

Selon un mode de réalisation, la diminution de la prise alimentaire concerne la période allant de l'injection à 3 heure après l'injection.

Selon un mode de réalisation, la diminution de la prise alimentaire concerne la période allant de l'injection à 2 heures après l'injection.

Selon un mode de réalisation, la diminution de la prise alimentaire concerne la période allant de l'injection à 1 heures après l'injection.

L'invention concerne également des formulations pharmaceutiques stables comprenant de telles compositions.

Les exigences permettant d'obtenir une formulation pharmaceutique injectable pour le traitement du diabète sont notamment :
- une formulation liquide aqueuse stable physiquement et chimiquement au moins deux semaines, voire un mois à 30°C (usages multiples) et au moins un an, voire 2 ans à 5°C,
- une compatibilité avec les conservateurs anti-microbiens.

De la même façon, les formulations d'insuline humaine A21G avec un analogue du GLP-1 ou un agoniste du récepteur au GLP-1, encore appelé GLP-1 RA, par exemple de l'exenatide ou du lixisenatide, à pH compris de 3,5 à 4,4 ont une stabilité physique et chimique permettant le développement d'une formulation liquide stable au moins deux semaines, voire un mois à 30°C et au moins un an, voire 2 ans, à 5°C

S'agissant de la stabilité, la méthode classique pour mesurer les stabilités des protéines ou peptides consiste à mesurer la formation de fibrilles à l'aide de Thioflavine T, encore appelée ThT. Cette méthode permet de mesurer dans des conditions de température et d'agitation permettant une accélération du phénomène, le temps de latence avant la formation de fibrilles par mesure de l'augmentation de la fluorescence. Les compositions selon l'invention ont un temps de latence avant la formation de fibrilles nettement supérieur à ceux décrits dans la littérature. Les compositions selon l'invention présentent une stabilité physique et chimique, bien supérieure à celles décrites dans l'art antérieur en utilisant des insulines prandiales commerciales.

Dans un mode de réalisation, les formulations selon l'invention présentent un temps de latence mesuré par ThT au moins égal à 8 heures.

Dans un mode de réalisation, les formulations selon l'invention présentent un temps de latence mesuré par ThT au moins égal à 10 heures.

Dans un mode de réalisation, les formulations selon l'invention présentent un temps de latence mesuré par ThT au moins égal à 15 heures.

Dans un mode de réalisation, les formulations selon l'invention présentent un temps de latence mesuré par ThT au moins égal à 20 heures.

Dans un mode de réalisation, les formulations selon l'invention présentent un temps de latence mesuré par ThT au moins égal à 25 heures.

Dans un mode de réalisation, l'invention concerne une composition sous forme d'une solution aqueuse injectable, dont le pH est compris de 3,5 à 4,4, comprenant au moins l'insuline humaine A21G et un agoniste au récepteur de l'amyline ou un analogue d'amyline. Selon un mode de réalisation il s'agit du pramlintide.

Dans un mode de réalisation, l'invention concerne une composition sous forme d'une solution aqueuse injectable, dont le pH est compris de 3,5 à 4,2, comprenant au moins l'insuline humaine A21G et un agoniste au récepteur de l'amyline ou un analogue d'amyline. Selon un mode de réalisation il s'agit du pramlintide.

Dans un mode de réalisation, l'invention concerne une composition sous forme d'une solution aqueuse injectable, dont le pH est compris de 3,8 à 4,2, comprenant au moins l'insuline humaine A21G et un agoniste au récepteur de l'amyline ou un analogue d'amyline. Selon un mode de réalisation il s'agit du pramlintide.

Dans un mode de réalisation, l'invention concerne une composition sous forme d'une solution aqueuse injectable, dont le pH est 4,0, comprenant au moins l'insuline humaine A21G et un agoniste au récepteur de l'amyline ou un analogue d'amyline. Selon un mode de réalisation il s'agit du pramlintide.

Dans un mode de réalisation, l'invention concerne une composition sous forme d'une solution aqueuse injectable, dont le pH est compris de 3,5 à 4,4, comprenant au moins l'insuline humaine A21G et un agoniste au récepteur du GLP-1 ou un analogue du GLP-1. Selon un mode de réalisation ledit agoniste au récepteur du GLP-1 est l'exénatide. Selon un autre mode de réalisation ledit agoniste au récepteur du GLP-1 est le lixisénatide.

Dans un mode de réalisation, l'invention concerne une composition sous forme d'une solution aqueuse injectable, dont le pH est compris de 3,5 à 4,2, comprenant au moins l'insuline humaine A21G et un agoniste au récepteur du GLP-1 ou un analogue du GLP-1. Selon un mode de réalisation ledit agoniste au récepteur du GLP-1 est l'exénatide. Selon un autre mode de réalisation ledit agoniste au récepteur du GLP-1 est le lixisénatide.

Dans un mode de réalisation, l'invention concerne une composition sous forme d'une solution aqueuse injectable, dont le pH est compris de 3,8 à 4,2, comprenant au moins l'insuline humaine A21G et un agoniste au récepteur du GLP-1 ou un analogue du GLP-1. Selon un mode de réalisation ledit agoniste au récepteur du GLP-1 est l'exénatide. Selon un autre mode de réalisation ledit agoniste au récepteur du GLP-1 est le lixisénatide.

Dans un mode de réalisation, l'invention concerne une composition sous forme d'une solution aqueuse injectable, dont le pH est 4,0, comprenant au moins l'insuline humaine A21G et un agoniste au récepteur du GLP-1 ou un analogue du GLP-1. Selon un mode de réalisation ledit agoniste au récepteur du GLP-1 est l'exénatide. Selon un autre mode de réalisation ledit agoniste au récepteur du GLP-1 est le lixisénatide.

Dans un mode de réalisation, l'invention concerne une composition sous forme d'une solution aqueuse injectable, dont le pH est compris de 3,5 à 4,4, comprenant au moins de l'insuline humaine A21G, un agoniste au récepteur de l'amyline ou un analogue d'amyline, et un agoniste au récepteur du GLP-1 ou un analogue du GLP-1. Selon un mode de réalisation ledit agoniste au récepteur du GLP-1 est l'exénatide. Selon un autre mode de réalisation ledit agoniste au récepteur du GLP-1 est le lixisénatide. Selon encore un autre mode de réalisation ledit agoniste au récepteur de l'amyline ou analogue d'amyline est le pramlintide.

Dans un mode de réalisation, l'invention concerne une composition sous forme d'une solution aqueuse injectable, dont le pH est compris de 3,5 à 4,2, comprenant au moins l'insuline humaine A21G, au moins un agoniste au récepteur de l'amyline ou un analogue d'amyline, et au moins un agoniste au récepteur du GLP-1 ou un analogue du GLP-1. Selon un mode de réalisation ledit agoniste au récepteur du GLP-1 est l'exénatide. Selon un autre mode de réalisation ledit agoniste au récepteur du GLP-1 est le lixisénatide. Selon encore un autre mode de réalisation ledit agoniste au récepteur de l'amyline ou analogue d'amyline est le pramlintide.

Dans un mode de réalisation, l'invention concerne une composition sous forme d'une solution aqueuse injectable, dont le pH est compris de 3,8 à 4,2, comprenant au moins de l'insuline humaine A21G, au moins un agoniste au récepteur de l'amyline ou un analogue d'amyline, et au moins un agoniste au récepteur du GLP-1 ou un analogue du GLP-1. Selon un mode de réalisation ledit agoniste au récepteur du GLP-1 est de l'exénatide. Selon un autre mode de réalisation ledit agoniste au récepteur du GLP-1 est du lixisénatide. Selon encore un autre mode de réalisation ledit agoniste au récepteur de l'amyline ou analogue d'amyline est le pramlintide.

Dans un mode de réalisation, l'invention concerne une composition sous forme d'une solution aqueuse injectable, dont le pH est 4,0, comprenant au moins de l'insuline humaine A21G, au moins un agoniste au récepteur de l'amyline ou un analogue d'amyline, et au moins un agoniste au récepteur du GLP-1 ou un analogue du GLP-1. Selon un mode de réalisation ledit agoniste au récepteur du GLP-1 est de l'exénatide. Selon un autre mode de réalisation ledit agoniste au récepteur du GLP-1 est du lixisénatide. Selon encore un autre mode de réalisation ledit agoniste au récepteur de l'amyline ou analogue d'amyline est le pramlintide.

Il est particulièrement intéressant de combiner en solution aqueuse l'insuline humaine A21G avec un analogue de l'amyline, un agoniste au récepteur de l'amyline ou le GLP-1 et avec un analogue du GLP-1, ou un agoniste au récepteur du GLP-1 car cette combinaison dite « triple » permet notamment de potentialiser les effets de chaque hormone et de réduire les doses de chacune d'elles.

Les compositions sous forme d'une solution aqueuse injectable selon l'invention sont des solutions limpides. On entend par « solution limpide », des compositions qui satisfont aux critères décrits dans les pharmacopées américaine et européenne concernant les solutions injectables. Dans la pharmacopée US, les solutions sont définies dans la partie <1151> faisant référence à l'injection (<1>) (faisant référence à <788> selon USP 35 et précisé dans <788> selon USP 35 et dans <787>, <788> et <790> USP 38 (à partir du 1^{er} aout 2014), selon USP 38). Dans la pharmacopée européenne, les solutions injectables doivent remplir les critères donnés dans les sections 2.9.19 et 2.9.20.

Dans la présente demande, l'amyline telle que mentionnée fait référence aux composés décrits dans les brevets US 5,124,314 et US 5,234,906. On entend par « analogue », lorsqu'il est utilisé par référence à un peptide ou une protéine, dans lequel un ou plusieurs résidus d'acides aminés constitutifs de la séquence primaire ont été substitués par d'autres résidus d'acides aminés et/ou dans lequel un ou plusieurs résidus d'acides aminés constitutifs ont été supprimés et/ou dans lequel un ou plusieurs résidus d'acides aminés constitutifs ont été ajoutés. Le pourcentage d'homologie admis pour la présente définition d'un analogue est de 50 %. Dans le cas de l'amyline, un analogue peut être par exemple dérivé de la séquence d'acide aminé primaire de l'amyline en substituant un ou plusieurs acides aminés naturels ou non naturels ou peptidomimétiques.

L'exenatide et le lixisénatide respectivement décrits dans les demandes US2004/0023871 et WO0104156 sont généralement considérés comme des agonistes au récepteur du GLP-1. Dans un mode de réalisation, le suppresseur de glucagon à action prandiale est le pramlintide (Symlin^{®}) commercialisé par la société ASTRAZENECA AB.

Dans un mode de réalisation, les GLP-1, analogues de GLP-1, ou GLP-1 RA sont dits « à action courte » ou « prandiaux ». On entend par « à action courte » ou « prandiaux », des GLP-1, analogues de GLP-1, ou GLP-1 RA, dont la demi-vie apparente d'élimination après injection sous-cutanée chez l'homme est inférieure 8 heures, en particulier inférieure à 5 heures, de préférence inférieure à 4 heures ou bien encore inférieure à 3 heures, comme par exemple l'exenatide et le lixisenatide.

Dans un mode de réalisation, les GLP-1, les analogues de GLP-1, ou les GLP-1 RA sont choisis dans le groupe constitué par l'exenatide (Byetta^{®}, ASTRAZENECA), le lixisenatide (Lyxumia^{®}, SANOFI), leurs analogues ou dérivés et leurs sels pharmaceutiquement acceptables.

Dans un mode de réalisation, le GLP-1, analogue de GLP-1, ou GLP-1 RA est l'exenatide ou Byetta^{®}, ses analogues ou dérivés et leurs sels pharmaceutiquement acceptables.

Dans un mode de réalisation, GLP-1, analogue de GLP-1, ou GLP-1 RA est le lixisenatide ou Lyxumia^{®}, ses analogues ou dérivés et leurs sels pharmaceutiquement acceptables.

Dans un mode de réalisation, la concentration d'insuline humaine A21G est comprise de 240 à 3000 µM ou 40 à 500 U/mL.

Dans un mode de réalisation, la concentration d'insuline humaine A21G est 600 µM ou 100 U/mL.

Dans un mode de réalisation, la concentration d'insuline humaine A21G est 1200 µM ou 200 U/mL.

Dans un mode de réalisation, la concentration d'insuline humaine A21G est 1800 µM ou 300 U/mL.

Dans un mode de réalisation, la concentration d'insuline humaine A21G est 2400 µM ou 400 U/mL.

Dans un mode de réalisation, la concentration d'insuline humaine A21G est 3000 µM ou 500 U/mL.

Dans la présente demande 100 U/ml d'insuline humaine A21G correspond à 3,5 mg/ml.

Dans un mode de réalisation, la concentration en pramlintide est comprise de 0,32 à 5 mg/mL.

Dans un mode de réalisation, la concentration en pramlintide est comprise de 0,4 à 3 mg/mL.

Dans un mode de réalisation, la concentration en pramlintide est comprise de 0,5 à 2 mg/mL.

Dans un mode de réalisation, la concentration en pramlintide est comprise de 0,5 à 1,5 mg/mL.

Dans un mode de réalisation, la concentration en pramlintide est comprise de 0,6 à 1 mg/mL.

Dans un mode de réalisation, la concentration en pramlintide est de 1,0 mg/ml.

Dans un mode de réalisation, la concentration en pramlintide est de 0,6 mg/ml.

Dans un mode de réalisation, la concentration en exenatide est comprise de 10 à 1000 µg/ml.

Dans un mode de réalisation, la concentration en exenatide est comprise de 10 à 500 µg/ml.

Dans un mode de réalisation, la concentration en exenatide est comprise de 20 à 400 µg/ml.

Dans un mode de réalisation, la concentration en exenatide est comprise de 20 à 300 µg/ml.

Dans un mode de réalisation, la concentration en exenatide est comprise de 30 à 300 µg/ml.Dans un mode de réalisation, la concentration en exénatide est comprise de 30 à 150 µg/ml.

Dans un mode de réalisation, la concentration en exenatide est comprise de 40 à 150 µg/ml.

Dans un mode de réalisation, la concentration en exénatide est comprise de 40 à 80 µg/ml.

Dans un mode de réalisation, la concentration en exenatide est de 50 µg/ml.

Dans un mode de réalisation, la concentration en lixisenatide est comprise de 20 à 1000 µg/ml.

Dans un mode de réalisation, la concentration en lixisenatide est comprise de 20 à 800 µg/ml.

Dans un mode de réalisation, la concentration en lixisenatide est comprise de 40 à 600 µg/ml.

Dans un mode de réalisation, la concentration en lixisenatide est comprise de 60 à 600 µg/ml.

Dans un mode de réalisation, la concentration en lixisenatide est comprise de 60 à 300 µg/ml.

Dans un mode de réalisation, la concentration en lixisenatide est comprise de 80 à 300 µg/ml.

Dans un mode de réalisation, la concentration en lixisenatide est comprise de 80 à 160 µg/ml.

Dans un mode de réalisation, la concentration en lixisenatide est de 100 µg/ml.

Dans un mode de réalisation, la concentration en exenatide, ses analogues ou dérivés et leurs sels pharmaceutiquement acceptables est comprise dans un intervalle de 0,01 à 1,0 mg pour 100 U d'insuline.

Dans un mode réalisation, la concentration en exenatide, ses analogues ou dérivés et leurs sels pharmaceutiquement acceptables est de 0,01 à 0,5 mg pour 100 U d'insuline.

Dans un mode réalisation, la concentration en exenatide, ses analogues ou dérivés et leurs sels pharmaceutiquement acceptables est de 0,02 à 0,4 mg pour 100 U d'insuline.

Dans un mode réalisation, la concentration en exenatide, ses analogues ou dérivés et leurs sels pharmaceutiquement acceptables est de 0,03 à 0,3 mg pour 100 U d'insuline.

Dans un mode réalisation, la concentration en exenatide, ses analogues ou dérivés et leurs sels pharmaceutiquement acceptables est de 0,03 à 0,2 mg pour 100 U d'insuline.

Dans un mode réalisation, la concentration en exenatide, ses analogues ou dérivés et leurs sels pharmaceutiquement acceptables est de 0,03 à 0,15 mg pour 100 U d'insuline.

Dans un mode réalisation, la concentration en exenatide, ses analogues ou dérivés et leurs sels pharmaceutiquement acceptables est de 0,05 mg pour 100 U d'insuline.

Dans un mode de réalisation, la concentration en lixisenatide, ses analogues ou dérivés et leurs sels pharmaceutiquement acceptables est comprise dans un intervalle de 0,01 à 1 mg pour 100 U d'insuline.

Dans un mode réalisation, la concentration en lixisenatide, ses analogues ou dérivés et leurs sels pharmaceutiquement acceptables est de 0,01 à 0,5 mg pour 100 U d'insuline.

Dans un mode réalisation, la concentration en lixisenatide, ses analogues ou dérivés et leurs sels pharmaceutiquement acceptables est de 0,02 à 0,4 mg pour 100 U d'insuline.

Dans un mode réalisation, la concentration en lixisenatide, ses analogues ou dérivés et leurs sels pharmaceutiquement acceptables est de 0,03 à 0,3 mg pour 100 U d'insuline.

Dans un mode réalisation, la concentration en lixisenatide, ses analogues ou dérivés et leurs sels pharmaceutiquement acceptables est de 0,04 à 0,2 mg pour 100 U d'insuline.

Dans un mode réalisation, la concentration en lixisenatide, ses analogues ou dérivés et leurs sels pharmaceutiquement acceptables est de 0,04 à 0,15 mg pour 100 U d'insuline.

Dans un mode réalisation, la concentration en lixisenatide, ses analogues ou dérivés et leurs sels pharmaceutiquement acceptables est de 0,1 mg pour 100 U d'insuline.

Dans un mode de réalisation, les compositions selon l'invention sont réalisées par mélange de solutions, d'analogues d'amyline ou d'agonistes au récepteur de l'amyline, et de solutions de GLP-1, d'analogue de GLP-1 ou de d'agoniste au récepteur du GLP-1 RA en ratios volumiques compris dans un intervalle de 10/90 à 90/10.

Dans un mode de réalisation, les compositions selon l'invention comprennent en outre des sels de zinc à une concentration comprise de 0 à 800 µM pour 100 U d'insuline.

Dans un mode de réalisation, les compositions selon l'invention comprennent en outre des sels de zinc à une concentration comprise de 0 à 500 µM pour 100 U d'insuline.

Dans un mode de réalisation, les compositions selon l'invention comprennent en outre des sels de zinc à une concentration comprise de 100 à 500 µM pour 100 U d'insuline.

Dans un mode de réalisation, les compositions selon l'invention comprennent en outre des sels de zinc à une concentration comprise de 200 à 400 µM pour 100 U d'insuline.

Dans un mode de réalisation, les compositions selon l'invention comprennent en outre des sels de zinc à une concentration de 300 µM pour 100 U d'insuline.

Dans un mode de réalisation, les compositions selon l'invention comprennent en outre des tampons.

Dans un mode de réalisation, les compositions selon l'invention comprennent un tampon choisi dans le groupe constitué par un tampon acétate de sodium et le Tris.

Dans un mode de réalisation, les compositions selon l'invention comprennent en outre des conservateurs.

Dans un mode de réalisation, les conservateurs sont choisis dans le groupe constitué par le m-crésol et le phénol, seuls ou en mélange.

Dans un mode de réalisation, la concentration des conservateurs est comprise de 10 à 50 mM.

Dans un mode de réalisation, la concentration des conservateurs est comprise de 10 à 40 mM.

Dans un mode de réalisation, les compositions selon l'invention comprennent en outre un tensioactif.

Dans un mode de réalisation, le tensioactif est choisi dans le groupe constitué par le Poloxamer 188, le Tween^{®} 20, encore appelé Polysorbate 20, et le Tween^{®} 80, encore appelé Polysorbate 80.

Dans un mode de réalisation la concentration Tween^{®} 20 varie de 5 à 50 µg/ml.

Dans un mode de réalisation la concentration Tween^{®} 20 varie de 5 à 25 µg/ml.

Dans un mode de réalisation la concentration Tween^{®} 20 est de 10 µM.

Les compositions selon l'invention peuvent en outre comprendre des additifs tels que des agents de tonicité.

Dans un mode de réalisation, les agents de tonicité sont choisis dans le groupe constitué par la glycérine, le chlorure de sodium, le mannitol et la glycine.

Dans un mode de réalisation, les compositions selon l'invention comprennent en outre un anti-oxydant.

Dans un mode de réalisation, l'anti-oxydant est la méthionine.

Dans un mode de réalisation, la composition pharmaceutique comprend en outre au moins un promoteur d'absorption choisi parmi les promoteurs d'absorption, les promoteurs de diffusion ou les agents vasodilatateurs, seuls ou en mélange.

Les promoteurs d'absorption incluent, sans se limiter, les surfactants, par exemple, les sels biliaires, les sels d'acides gras ou les phospholipides ; les agents nicotiniques, comme les nicotinamides, les acides nicotiniques, la niacine, la niacinamide, la vitamine B3 et leurs sels ; les inhibiteurs de la trypsine pancréatique ; les sels de magnésium ; les acides gras polyinsaturés ; le phosphatidylcholine didécanoyl ; les aminopolycarboxylates ; la tolmétine ; le caprate de sodium ; l'acide salicylique ; l'acide oléique ; l'acide linoléique ; l'acide eicosapentaénoïque (EPA) ; l'acide docosahexaénoïque (DHA) ; l'acide benzylique ; les donneurs de monoxyde d'azote, par exemple, la 3-(2-Hydroxy-1-(1-méthyléthyl)-2-nitrosohydrazino)-1-propanamine, la *N*-éthyl-2-(1-éthyl-hydroxy-2-1-nitrosohydrazino)-éthanamine, ou la S-nitroso-*N*-acétylpenicillamine ; les acides biliaires, la glycine sous sa forme conjuguée à un acide biliaire ; l'ascorbate de sodium, l'ascorbate de potassium ; le salicylate de sodium, le salicylate de potassium, l'acide acétyl-salicylique, l'acide salicylosalicylique, l'acétylsalicylate d'aluminum, le salicylate de choline, le salicylamide, l'acétylsalicylate de lysine ; l'exalamide ; le diflunisal ; l'éthenzamide ; l'EDTA ; seul ou en mélange.

Dans un mode de réalisation, la composition pharmaceutique comprend en outre au moins un promoteur de diffusion. Des exemples de promoteur de diffusion incluent, sans se limiter, les glycosaminoglycanases, par exemple la hyaluronidase.

Dans un mode de réalisation, la composition pharmaceutique comprend en outre au moins un agent vasodilatateur.

Dans un mode de réalisation, la composition pharmaceutique comprend en outre au moins un agent vasodilatateur provoquant une hyperpolarisation en bloquant les canaux ioniques de calcium.

Dans un mode de réalisation, l'agent vasodilatateur provoquant une hyperpolarisation en bloquant les canaux ioniques de calcium est l'adénosine, un agent hyperpolarisant dérivé de l'endothélium, un inhibiteur de la phosphodiestérase de type 5 (PDE5), un agent d'ouverture des canaux potassiques ou toute combinaison de ces agents.

Dans un mode de réalisation, la composition pharmaceutique comprend en outre au moins un agent vasodilatateur à médiation par AMPc.

Dans un mode de réalisation, la composition pharmaceutique comprend en outre au moins un agent vasodilatateur à médiation par GMPc.

Dans un mode de réalisation, la composition pharmaceutique comprend en outre au moins un agent vasodilatateur choisi dans le groupe comprenant les agents vasodilatateurs qui agissent en provoquant une hyperpolarisation en bloquant les canaux ioniques de calcium, les agents vasodilatateurs à médiation par AMPc, et les agents vasodilatateurs à médiation par GMPc.

Le au moins un agent vasodilatateur est choisi dans le groupe comprenant, les donneurs de monoxyde d'azote, par exemple, la nitroglycérine, le dinitrate d'isosorbide, le mononitrate d'isosorbide, le nitrate d'amyl, l'érythrityle, le tétranitrate, et le nitroprussiate) ; la prostacycline et ses analogues, par exemple l'époprosténol sodique, l'iloprost, l'époprostenol, le tréprostinil ou le selexipag ; l'histamine, la 2-méthylhistamine, la 4-méthylhistamine; la 2-(2-pyridyl)éthylamine, la 2-(2-thiazolyl)éthylamine ; la papavérine, le chlorhydrate de papavérine ; le minoxidil ; la dipyridamole ; l'hydralazine ; l'adénosine, l'adénosine triphosphate; l'uridine trisphosphate ; le GPLC ; la L-carnitine ; l'arginine ; la prostaglandine D2 ; les sels de potassium ; et dans certains cas, les antagonistes des récepteurs α1 et α2 , par exemple, le prazosine, la phénoxybenzamine, la phentolamine, la dibénamine, le chlorhydrate de moxisylyte et la tolazoline), le bétazole, le dimaprit ; les agonistes des récepteurs β2, par exemple, l'isoprotérénol, la dobutamine, l'albutérol, la terbutaline, l'aminophylline, la théophylline, la caféine ; l'alprostadil, l'ambrisentan ; la cabergoline ; la diazoxide ; le mesilate de dihydralazine ; le chlorhydrate de diltiazem ; l'énoximone ; le chlorhydrate de flunarizine ; l'extrait de Ginkgo biloba ; le lévosimendan ; la molsidomine ; l'oxalate acide de naftidrofuryl ; le nicorandil ; la pentoxifylline ; le chlorure de phenoxybenzamine ; le piribédil base ; le mesilate de piribédil ; le regadenoson monohydrate ; le riociguat ; le sildenafil citrate, le tadalafil, le chlorhydrate trihydraté de vardenafil ; le chlorhydrate de trimetazidine ; la trinitrine ; le chlorhydrate de vérapamil ; les antagonistes des récepteur à l'endothéline, par exemple l'avanafil et le bosentran monohydrate ; et les inhibiteurs des canaux calciques, par exemple, l'amlodipine, l'aranidipine, l'azelnidipine, la barnidipine, la benidipine, la cilnidipine, la clévidipine, l'isradipine, l'efonidipine, la felodipine, la lacidipine, la lercanidipine, la manidipine, la nicardipine, la nifedipine, la nilvadipine, la nimodipine, la nisoldipine, la nitrendipine, la prandipine ; seul ou en mélange.

Dans un mode de réalisation, la composition selon l'invention contient de 3,5 mg/mL à 10,5 mg/mL d'insuline humaine A21G, 0,6 mg/mL à 3 mg/mL de pramlintide, 25 mM de m-crésol, 184 mM de glycérine à un pH de 4,0. Cette composition peut contenir en outre de 300 à 900 µM de zinc. Cette composition peut en outre comprendre du polysorbate 20, en particulier de 8 à 10 µM, et tout particulièrement de 8 µM.

Dans un mode de réalisation, la composition selon l'invention contient 3,5 mg/mL d'insuline humaine A21G, 0,6 à 1 mg/mL de pramlintide, 25 à 30 mM de m-crésol, 150 à 200 mM de glycérine, à un pH de 4,0. Cette composition peut contenir en outre 300 µM de zinc. Cette composition peut en outre comprendre du polysorbate 20, en particulier de 8 à 10 µM, et tout particulièrement de 8 µM.

Dans un mode de réalisation, la composition selon l'invention contient 3,5 mg/mL d'insuline humaine A21G, 0,6 mg/mL de pramlintide, 25 mM de m-crésol, 184 mM de glycérine, à un pH de 4,0. Cette composition peut contenir en outre 300 µM de zinc. Cette composition peut en outre comprendre du polysorbate 20, en particulier 10 µM.

Dans un mode de réalisation, la composition selon l'invention contient 3,5 mg/mL d'insuline humaine A21G, 0,6 mg/mL de pramlintide, 25 mM de m-crésol, 184 mM de glycérine, à un pH de 4,0. Cette composition peut contenir en outre 300 µM de zinc. Cette composition peut en outre comprendre du polysorbate 20, en particulier 8 µM.

Dans un mode de réalisation, la composition selon l'invention contient 3,5 mg/mL d'insuline humaine A21G, 1,0 mg/mL de pramlintide, 25 mM de m-crésol, 184 mM de glycérine, à un pH de 4,0. Cette composition peut contenir en outre 300 µM de zinc. Cette composition peut en outre comprendre du polysorbate 20, en particulier 8 µM.

Dans un mode de réalisation, la composition selon l'invention contient 7,0 mg/mL d'insuline humaine A21G, de 1,2 à 2,0 mg/mL de pramlintide, 25 mM de m-crésol, 150 à 200 mM de glycérine, à un pH de 4,0. Cette composition peut contenir en outre 600 µM de zinc. Cette composition peut en outre comprendre du polysorbate 20, en particulier de 8 à 10 µM, et tout particulièrement de 8 µM.

Dans un mode de réalisation, la composition selon l'invention contient 7,0 mg/mL d'insuline humaine A21G, 1,2 mg/mL de pramlintide, 25 mM de m-crésol, 184 mM de glycérine, à un pH de 4,0. Cette composition peut contenir en outre 600 µM de zinc. Cette composition peut en outre comprendre du polysorbate 20, en particulier 10 µM.

Dans un mode de réalisation, la composition selon l'invention contient 7,0 mg/mL d'insuline humaine A21G, 1,2 mg/mL de pramlintide, 25 mM de m-crésol, 184 mM de glycérine, à un pH de 4,0. Cette composition peut contenir en outre 600 µM de zinc. Cette composition peut en outre comprendre du polysorbate 20, en particulier 8 µM.

Dans un mode de réalisation, la composition selon l'invention contient 7,0 mg/mL d'insuline humaine A21G, 2,0 mg/mL de pramlintide, 25 mM de m-crésol, 184 mM de glycérine, à un pH de 4,0. Cette composition peut contenir en outre 600 µM de zinc. Cette composition peut en outre comprendre du polysorbate 20, en particulier 8 µM.

Dans un mode de réalisation, la composition selon l'invention contient 10,5 mg/mL d'insuline humaine A21G, 1,8 à 3 mg/mL de pramlintide, 25 mM de m-crésol, 150 à 200 mM de glycérine, à un pH de 4,0. Cette composition peut contenir en outre 900 µM de zinc. Cette composition peut en outre comprendre du polysorbate 20, en particulier de 8 à 10 µM, et tout particulièrement de 8 µM.

Dans un mode de réalisation, la composition selon l'invention contient 10,5 mg/mL d'insuline humaine A21G, 1,8 mg/mL de pramlintide, 25 mM de m-crésol, 184 mM de glycérine, à un pH de 4,0. Cette composition peut contenir en outre 900 µM de zinc. Cette composition peut en outre comprendre du polysorbate 20, en particulier 10 µM.

Dans un mode de réalisation, la composition selon l'invention contient 10,5 mg/mL d'insuline humaine A21G, 1,8 mg/mL de pramlintide, 25 mM de m-crésol, 184 mM de glycérine, à un pH de 4,0. Cette composition peut contenir en outre 900 µM de zinc. Cette composition peut en outre comprendre du polysorbate 20, en particulier 8 µM.

Dans un mode de réalisation, la composition selon l'invention contient 10,5 mg/mL d'insuline humaine A21G, 3 mg/mL de pramlintide, 25 mM de m-crésol, 184 mM de glycérine, à un pH de 4,0. Cette composition peut contenir en outre 900 µM de zinc. Cette composition peut en outre comprendre du polysorbate 20, en particulier 8 µM.

Les compositions selon l'invention peuvent comprendre en outre tous les excipients conformes aux Pharmacopées, notamment EP et/ou US, et compatibles avec les insulines utilisées aux concentrations d'usage.

Selon un mode de réalisation, la composition peut être sous forme solide ou lyophilisée. Cette composition peut alors servir à reconstituer une solution ou une formulation.

Les modes d'administration envisagés sont par voie intraveineuse, sous-cutanée, intradermique ou intramusculaire.

Selon un mode de réalisation particulier le mode d'administration est la voie sous-cutanée.

Les voies d'administration transdermique, orale, nasale, vaginale, oculaire, buccale, pulmonaire sont également envisagées.

L'invention concerne également une pompe, implantable ou transportable, comprenant une composition selon l'invention.

L'invention concerne encore l'utilisation d'une composition selon l'invention destinée à être placée dans une pompe, implantable ou transportable.

L'invention concerne également des formulations unidoses.

Dans un mode de réalisation, les formulations sont sous forme d'une solution injectable.

La préparation d'une composition selon l'invention présente l'avantage de pouvoir être réalisée par simple mélange d'une solution aqueuse, d'un analogue d'amyline ou d'un agoniste au récepteur de l'amyline, et d'insuline humaine A21G, en solution aqueuse ou sous forme lyophilisée.

Si nécessaire, la composition du mélange est ajustée en excipients tels que glycérine, m-crésol, chlorure de zinc, et polysorbate 20 (Tween^{®} 20). Cette addition peut être effectuée par ajout de solutions concentrées desdits excipients.

Dans un mode de réalisation, les compositions sont caractérisées en ce que lesdites compositions présentent une solubilité à un pH de 4,0 et une stabilité physique mesurée par ThT supérieure à celle d'une composition de référence comprenant un analogue d'amyline ou un agoniste au récepteur de l'amyline et une insuline prandiale commerciale.

La ThT est mesuré selon le protocole décrit dans les exemples.

Dans un mode de réalisation, les compositions sont caractérisées en ce que lesdites compositions présentent une solubilité à un pH de 4,0 et une stabilité physique mesurée par ThT supérieure à celle d'une composition de référence comprenant un GLP-1, un analogue de GLP-1 ou un agoniste de récepteur de GLP-1 et une insuline prandiale commerciale.

L'insuline et les insulines analogues peuvent être obtenues par des méthodes de technologies de l'ADN recombinante en bactéries telle que *l'Escherichia Coli* ou en levures telle que le *Saccharomyces Cerevisiae* (voir par exemple G. Walsh Appl. Microbiol. Biotechnol. 2005, 67, 151-159). Généralement, on produit une proinsuline qui est ensuite digérée par des enzymes telles que la trypsine et la carboxypeptidase B pour obtenir la séquence souhaitée.

Pour la fabrication d'insuline humaine A21G, on code la proinsuline pour avoir la glycine en A21 et après digestion par la trypsine et la carboxypetidase B, on obtient l'insuline souhaitée. Un mode opératoire est décrit par Kohn et al., dans Peptides 2007, 28, 935-948.

Il est aussi divulgué à titre illustratif un procédé d'obtention de l'insuline humaine A21G comprenant au moins une étape consistant à faire réagir l'insuline humaine A21G, B31R, B32R (insuline glargine) avec la carboxypeptidase B de rat à un ratio insuline/carboxypeptidase compris entre 500 et 2000, à un pH compris de 7,5 à 8,5 et à une température comprise de 20 à 30°C pendant 10 à 20 heures. Le produit peut ensuite être purifié. Cette purification peut être effectuée par par chromatographie liquide.

L'insuline humaine A21G peut donc être obtenue en enlevant les deux arginines de l'insuline glargine par digestion avec une carboxypeptidase B. Après digestion enzymatique, l'insuline humaine A21G est purifiée par chromatographie puis isolée par lyophilisation ou par cristallisation par des méthodes classiques.

### Description des figures :

**Figure 1****:** Détermination graphique du temps de latence de fibrillation.
   A la figure 1 est représentée la détermination graphique du temps de latence de fibrillation sur un exemple virtuel. En abscisse figure le temps en minutes, en ordonnée figure la fluorescence ThT en unité arbitraire (u.a.) et LT figure le temps de latence, ou « lag time ».
**Figure 2** : Concentrations en pramlintide et insuline plasmatique après administration de la formulation A21-8 (moyenne ± erreur standard)
   Les carrés représentent la concentration en insuline et les triangles la concentration en pramlintide.
   En abscisse figure le temps en minutes après injection, en ordonnée à gauche la concentration en insuline en pmol/l et en ordonnée la concentration en pramlintide en pmol/l corrigées de la ligne de base, ou « baseline ».

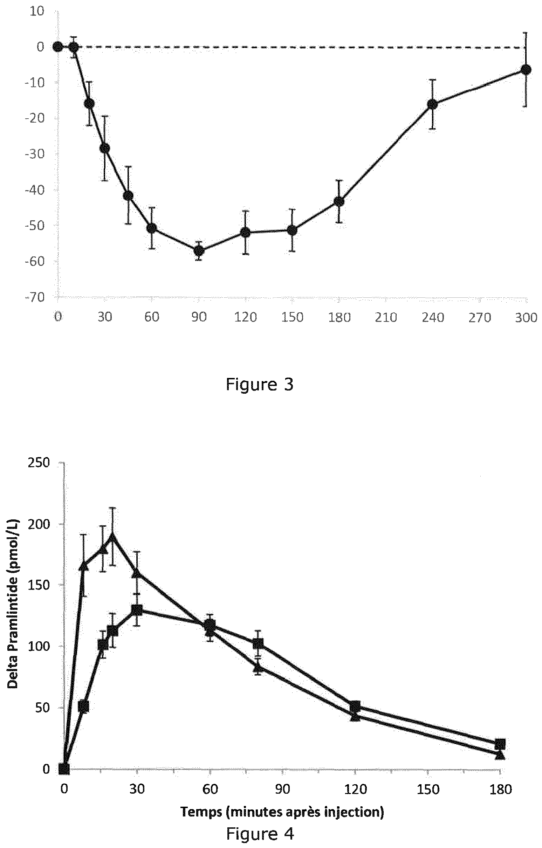

**Figure 3** : Glycémie après administration de la formulation A21-8 (moyenne ± erreur standard).
   En abscisse le temps en minutes après injection, et en ordonnée la glycémie en % du niveau basal.
**Figure 4** : Concentrations en pramlintide après administration des formulation A21-9 (courbe tracée avec les carrés) et PRAM (courbe tracée avec les triangles) (moyenne ± erreur standard).
   En abscisse le temps en minutes après injection, et en ordonnée les concentrations en pramlintide corrigées de la ligne de base, ou « baseline » (concentrations pré-doses retranchées individuellement), en pmol/L.

### Exemples

### Exemple 1. Préparation de l'insuline humaine A21G

On mélange 5 g d'insuline glargine (Gan & Lee Pharmaceuticals) à de l'enzyme carboxypeptidase B (Référence 08039852001 ; Sigma-Aldrich) à pH 8,0 (pH ajusté par ajout de tampon Tris) et on laisse à 25°C pendant 17 heures, la concentration en insuline glargine étant d'environ 4 mg/mL. Le ratio enzyme/glargine est de 1/500. Le mélange est ensuite purifié par chromatographie liquide, dialysé contre de l'acide chlorhydrique 0,01N puis lyophilisé. On obtient l'insuline humaine A21G avec une pureté de 98% et un rendement d'environ 90%. La masse molaire de l'insuline mesurée par spectrométrie de masse (Maldi-Tof) est de 5752 Da. L'insuline humaine A21G peut aussi être obtenue à partir de la technologie recombinante comme décrit par Kohn et al. (Peptides 2007, 28, 935-948).

**Exemple 2.** Compositions d'insulines prandiales et de pramlintide, d'exénatide ou de lixisénatide à pH acide.

### Préparation d'une solution d'insuline humaine A21G 100 U/mL (3,5 mg/ml) et de pramlintide 1 mg/mL contenant du m-crésol (25 mM), de la glycérine (184 mM) et du chlorure de zinc (300 µM) à pH acide de 3,5 ou 4,0.

Une solution concentrée d'excipients (m-crésol, glycérine) est ajoutée à une solution d'insuline humaine A21G concentrée (300 U/mL à pH 3,5). Une solution de pramlintide (Ambiopharm) concentrée (10 mg/mL à pH 4) et une solution de concentrée de chlorure de zinc sont ajoutées à cette solution concentrée d'insuline humaine A21G et d'excipients de manière à obtenir la composition finale visée. Le pH final, soit 3,5 ou 4,0, est ajusté à la valeur désirée par ajout d'une solution aqueuse de NaOH ou d'HCI. La solution obtenue est limpide et homogène, celle-ci est filtrée sur 0,22 µm et stockée dans des cartouches en verre (1 mL de solution par cartouche).

### Préparation d'une solution d'insuline humaine A21G 100 U/mL et de pramlintide 0,6 mg/mL contenant du m-crésol (25 mM), de la glycérine (184 mM) et du chlorure de zinc (300 µM) à pH acide de 4,0.

Cette solution est préparée de la même manière que la solution présentée ci-dessus.

### Préparation d'une solution d'insuline humaine A21G 100 U/mL et de pramlintide 1 mg/mL contenant du m-crésol (25 mM) et de la glycérine (184 mM) à pH acide de 4,0.

Une solution concentrée d'excipients (m-crésol, glycérine) est ajoutée à une solution d'insuline humaine A21G concentrée (800 U/mL à pH 3,5). Une solution de pramlintide concentrée (10 mg/mL à pH 4) est ajoutée à cette solution concentrée d'insuline humaine et d'excipients de manière à obtenir la composition finale visée. Le pH final, soit 4,0, est ajusté à la valeur désirée par ajout d'une solution aqueuse de NaOH ou d'HCI. La solution obtenue est limpide et homogène, celle-ci est filtrée sur 0,22 µm et stockée dans des cartouches en verre (1 mL de solution par cartouche).

### Préparation d'une solution d'insuline humaine A21G 100 U/mL et de pramlintide 1 mg/mL contenant du m-crésol (25 mM), de la glycérine (184 mM) et du Tween 20 (10 µg/ml) à pH 4.

Une solution concentrée d'excipients (m-crésol, glycérine) est ajoutée à une solution d'insuline humaine A21G concentrée (300 U/mL à pH 3,5). Une solution de pramlintide concentrée (10 mg/mL à pH 4) et une solution de tween 20 concentrée sont ajoutées à cette solution concentrée d'insuline humaine A21G et d'excipients de manière à obtenir la composition finale visée. Le pH final est ajusté à la valeur désirée par ajout d'une solution aqueuse de NaOH ou d'HCI. La solution obtenue est limpide et homogène, celle-ci est filtrée sur 0,22 µm et stockée dans des cartouches en verre (1 mL de solution par cartouche).

### Préparation d'une solution d'insuline humaine A21G 100 U/mL et de pramlintide 1 mg/mL contenant du m-crésol (25 mM), de la glycérine (184 mM), du chlorure de zinc (300 µM) et du Tween 20 (10 µg/ml) à pH 4.

Une solution concentrée d'excipients (m-crésol, glycérine est ajoutée à une solution d'insuline humaine A21G concentrée (300 U/mL à pH 3,5). Une solution de pramlintide concentrée (10 mg/mL à pH 4), une solution de chlorure de zinc concentrée et une solution de tween 20 concentrée sont ajoutées à cette solution concentrée d'insuline humaine A21G et d'excipients de manière à obtenir la composition finale visée. Le pH final est ajusté à la valeur désirée par ajout d'une solution aqueuse de NaOH ou d'HCI. La solution obtenue est limpide et homogène, celle-ci est filtrée sur 0,22 µm et stockée dans des cartouches en verre (1 mL de solution par cartouche).

### Préparation d'une solution d'insuline humaine A21G 100 U/mL et d'exénatide 50 µg/mL contenant du m-crésol (25 mM), de la glycérine (184 mM) et du chlorure de zinc (300 µM) à pH acide.

Une solution concentrée d'excipients (m-crésol, glycérine)) est ajoutée à une solution d'insuline humaine A21G concentrée (300 U/mL à pH 3,5). Une solution d'exénatide (Bachem) concentrée (10,5 mg/mL à pH 4) et une solution concentrée de chlorure de zinc sont ajoutées à cette solution concentrée d'insuline humaine A21G et d'excipients de manière à obtenir la composition finale visée. Le pH final 4,0 est ajusté à la valeur désirée par ajout d'une solution aqueuse de NaOH ou d'HCI. La solution obtenue est limpide et homogène, celle-ci est filtrée sur 0,22 µm et stockée dans des cartouches en verre (1 mL de solution par cartouche).

### Préparation d'une solution d'insuline humaine A21G 100 U/mL et de lixisénatide 100 µg/mL contenant du m-crésol (25 mM), de la glycérine (184 mM) et du chlorure de zinc (300 µM) à pH acide.

Une solution concentrée d'excipients (m-crésol, glycérine) est ajoutée à une solution d'insuline humaine A21G concentrée (230 U/mL à pH 3,5). Une solution de de lixisénatide (Ambiopharm) concentrée (10,5 mg/mL à pH 4) et une solution concentrée de chlorure de zinc sont ajoutées à cette solution concentrée d'insuline humaine A21G et d'excipients de manière à obtenir la composition finale visée. Le pH final 4,0 est ajusté à la valeur désirée par ajout d'une solution aqueuse de NaOH ou d'HCI. La solution obtenue est limpide et homogène, celle-ci est filtrée sur 0,22 µm et stockée dans des cartouches en verre (1 mL de solution par cartouche).

### Préparation d'une solution d'insuline humaine A21G 100 U/mL, d'exénatide 50 µg/mL et de pramlintide 0,6 mg/mL contenant du m-crésol (25 mM), de la glycérine (184 mM) et du Tween 20 (10 µg/ml) à pH acide de 4,0.

Une solution concentrée d'excipients (m-crésol, glycérine) est ajoutée à une solution d'insuline humaine A21G concentrée (300 U/mL à pH 3,5). Une solution de pramlintide (Ambiopharm) concentrée (10 mg/mL à pH 4), une solution d'exénatide (Bachem) concentrée (10,5 mg/mL à pH 4) et une solution de tween 20 concentrée sont ajoutées à cette solution concentrée d'insuline humaine A21G et d'excipients de manière à obtenir la composition finale visée. Le pH final 4,0, est ajusté à la valeur désirée par ajout d'une solution aqueuse de NaOH ou d'HCI. La solution obtenue est limpide et homogène, celle-ci est filtrée sur 0,22 µm et stockée dans des cartouches en verre (1 mL de solution par cartouche).

### Préparation d'une solution d'insuline humaine 100 U/mL et de pramlintide 1 mg/mL contenant du m-crésol (25 mM), de la glycérine (184 mM) et du chlorure de zinc (300 µM) à pH acide de 3,5 ou 4,0.

Une solution concentrée d'excipients (m-crésol, glycérine) est ajoutée à une solution d'insuline humaine (Amphastar Pharmaceuticals) concentrée (800 U/mL à pH 3,5). Une solution de pramlintide concentrée (10 mg/mL à pH 4) et une solution concentrée de chlorure de zincsont ajoutées à cette solution concentrée d'insuline humaine et d'excipients de manière à obtenir la composition finale visée. Le pH final, soit 3,5 ou 4,0, est ajusté à la valeur désirée par ajout d'une solution aqueuse de NaOH ou d'HCI. La solution obtenue est limpide et homogène, celle-ci est filtrée sur 0,22 µm et stockée dans des cartouches en verre (1 mL de solution par cartouche).

### Préparation d'une solution d'insuline aspart 100 U/mL et de pramlintide 1 mg/mL contenant du m-crésol (25 mM), de la glycérine (184 mM) et du chlorure de zinc (300 µM) à pH acide de 3,5 ou 4,0.

Une solution concentrée d'excipients (m-crésol, glycérine) est ajoutée à une solution d'insuline aspart (HEC Pharmaceuticals) concentrée (500 U/mL à pH 3). Une solution de pramlintide concentrée (10 mg/mL à pH 4) et une solution concentrée de chlorure de zincsont ajoutées à cette solution concentrée d'insuline aspart et d'excipients de manière à obtenir la composition finale visée. Le pH final, soit 3,5 ou 4,0, est ajusté à la valeur désirée par ajout d'une solution aqueuse de NaOH ou d'HCI. La solution ajustée à pH 4,0 est turbide dès l'ajustement de pH. La solution ajustée à pH 3,5 est limpide. Celle-ci est filtrée sur 0,22 µm et stockée dans des cartouches en verre (1 mL de solution par cartouche).

### Préparation d'une solution d'insuline lispro 100 U/mL et de pramlintide 1 mg/mL contenant du m-crésol (25 mM), de la glycérine (184 mM) et du chlorure de zinc (300 µM) à pH acide de 3,5 ou 4,0.

Une solution concentrée d'excipients (m-crésol, glycérine) est ajoutée à une solution d'insuline lispro (Gan et Lee Pharmaceuticals) concentrée (650 U/mL à pH 3). Une solution de pramlintide concentrée (10 mg/mL à pH 4) et une solution concentrée de chlorure de zinc sont ajoutéesà cette solution concentrée d'insuline lispro et d'excipients de manière à obtenir la composition finale visée. Le pH final, soit 3,5 ou 4,0, est ajusté à la valeur désirée par ajout d'une solution aqueuse de NaOH ou d'HCI. La solution obtenue est limpide et homogène, celle-ci est filtrée sur 0,22 µm et stockée dans des cartouches en verre (1 mL de solution par cartouche).

### Préparation d'une solution d'insuline glulisine 100 U/mL et de pramlintide 1 mg/mL contenant les excipients du produit commercial Apidra^{®} (29 mM m-crésol, 50 mM de Tris, 86 mM de chlorure de zinc et 8,15 µM de Tween 20 à pH acide de 3,0, 3,5 ou 4,0.

Le pH de la solution commerciale d'insuline glulisine, Apidra^{®}, est ajusté à pH 2,5 par ajout d'une solution aqueuse d'HCI. Cette solution est ajoutée à pramlintide sous la forme de poudre de manière à obtenir une solution contenant 100 U/mL d'insuline et 1 mg/mL de pramlintide. Le pH final est ajusté à la valeur désirée par ajout d'une solution aqueuse de NaOH ou d'HCI. Les solutions ajustées à pH 3,5 et 4,0 sont turbides dès l'ajustement de pH. La solution ajustée à pH 3,0 est limpide. Celle-ci est filtrée sur 0,22 µm et stockée dans des cartouches en verre (1 mL de solution par cartouche). Après quelques heures de stockage la solution est turbide et hétérogène.

### Préparation d'une solution de pramlintide 1 mg/mL contenant du m-crésol (20 mM), du mannitol (236 mM) et du tampon acide acétique/acétate de sodium (30 mM) à pH 4,0.

Une solution de pramlintide concentrée à 10 mg/mL est préparée par dissolution de pramlintide sous forme de poudre achetée chez Ambiopharm. Cette solution est ajoutée à une solution concentrée d'excipients (m-crésol, mannitol, tampon acide acétique/acétate de sodium) de manière à obtenir la composition finale visée. Le pH final est ajusté à 4,0 ± 0,2 par ajout de NaOH/HCI.

### Préparation d'une solution d'insuline humaine A21G 100 U/mL et de pramlintide 0.6 mg/mL contenant du m-crésol (25 mM), de la glycérine (184 mM), du tampon acide acétique/acétate de sodium (18 mM) et du Tween 20 (8 µM) à pH 4.

Des solutions concentrées de glycérine et m-crésol sont ajoutées à une solution d'insuline humaine A21G concentrée dans un tampon acide acétique/acétate de sodium à pH 4 (300 U/mL à pH 4). Une solution de pramlintide (Ambiopharm) concentrée (10 mg/mL à pH 4) et une solution concentrée de Tween 20 sont enfin ajoutées à cette solution concentrée d'insuline humaine A21G et d'excipients de manière à obtenir la composition finale visée. Le pH final est ajusté à la valeur désirée par ajout d'une solution aqueuse de NaOH ou d'HCI. La solution obtenue est limpide et homogène, celle-ci est filtrée sur 0,22 µm.

Les compositions préparées ci-dessus sont présentées dans le tableau 1 ci-dessous :

**Tableau 1 : compositions d'insuline et/ou de suppresseurs de glucagon**

| **Composition s** | **Type d'Insulin e (U/ml)** | **Pramlintid e (mg/ml)** | **pH** | **Zinc (µM )** | **Tween 20 (µg/ml )** | **GLP-1 RA** | **Autres excipient s** |
|---|---|---|---|---|---|---|---|
| A21-1 | Insuline humaine A21G (100) | 1,0 | 4, 0 | 300 | 0 | 0 | m-crésol (25 mM) glycérine (184 mM) |
| A21-2 | Insuline humaine A21G (100) | 1,0 | 4, 0 | 0 | 0 | 0 | m-crésol (25 mM) glycérine (184 mM) |
| A21-3 | Insuline humaine A21G (100) | 1,0 | 3, 5 | 300 | 0 | 0 | m-crésol (25 mM) glycérine (184 mM) |
| A21-4 | Insuline humaine A21G (100) | 1,0 | 4, 0 | 0 | 10 | 0 | m-crésol (25 mM) glycérine (184 mM) |
| A21-5 | Insuline humaine A21G (100) | 1,0 | 4, 0 | 300 | 10 | 0 | m-crésol (25 mM) glycérine (184 mM) |
| A21-6 | Insuline humaine A21G (100) | 0 | 4, 0 | 300 | 0 | exénatide 50 µg/ml | m-crésol (25 mM) glycérine (184 mM) |
| A21-7 | Insuline humaine A21G (100) | 0 | 4, 0 | 300 | 0 | lixisénatid e 100 µg/ml | m-crésol (25 mM) glycérine (184 mM) |
| A21-8 | Insuline humaine A21G (100) | 0,6 | 4, 0 | 300 | 0 | 0 | m-crésol (25 mM) glycérine (184 mM) |
| RHI-1 | Insuline humaine (100) | 1,0 | 4,0 | 300 | 0 | 0 | m-crésol (25 mM) glycérine (184 mM) |
| RHI-2 | Insuline humaine (100) | 1,0 | 3,5 | 300 | 0 | 0 | m-crésol (25 mM) glycérine (184 mM) |
| ASP-1 | Insuline aspart (100) | 1,0 | 4,0 | 300 | 0 | 0 | m-crésol (25 mM) glycérine (184 mM) |
| ASP-2 | Insuline aspart (100) | 1,0 | 3,5 | 300 | 0 | 0 | m-crésol (25 mM) glycérine (184 mM) |
| GLU-1 | Insuline glulisine (100) | 1,0 | 4,0 | 300 | 10 | 0 | m-crésol (29 mM) Tris (50 mM) NaCl (86 mM) |
| GLU-2 | Insuline glulisine (100) | 1,0 | 3,5 | | 10 | 0 | m-crésol (29 mM Tris (50 mM) NaCl (86 mM) |
| GLU-3 | Insuline glulisine (100) | 1,0 | 3,0 | | 10 | 0 | m-crésol (29 mM) Tris (50 mM) NaCl (86 mM) |
| LIS-1 | Insuline lispro (100) | 1,0 | 4,0 | 300 | 0 | 0 | m-crésol (25 mM) glycérine (184 mM) |
| LIS-2 | Insuline lispro (100) | 1,0 | 3,5 | 300 | 0 | 0 | m-crésol (25 mM) glycérine (184 mM) |
| PRAM | - | 1,0 | 4,0 | - | - | 0 | m-crésol (20 mM) mannitol (236 mM) acétate (30 mM) |
| A21-9 | Insuline humaine A21G (100) | 0,6 | 4,0 | - | 10 | 0 | m-crésol (25 mM) glycérine (184 mM) acétate (18 mM) |
| A21-10 | Insuline humaine A21G (100) | 0,6 | 4,0 | - | 10 | exénatide 50 µg/ml | m-crésol (25 mM) glycérine (184 mM) |

**Exemple 3.** Etude de compatibilité des insulines prandiales avec le pramlintide à pH acide.

### Aspect visuel des solutions d'insuline et de pramlintide à pH acide.

L'observation est réalisée à température ambiante après 2 à 3 heures de stabilisation de la solution stockée en cartouches. Le **Tableau 2** présente l'aspect visuel de solutions d'insuline et de pramlintide décrites précédemment.

**Tableau 2 : Aspect visuel des solutions d'insuline et de pramlintide,**

| **Composition** | **Aspect visuel** |
|---|---|
| A21-1 | Limpide |
| A21-2 | Limpide |
| A21-3 | Limpide |
| RHI-1 | Limpide |
| RHI-2 | Limpide |
| ASP-1 | Turbide |
| ASP-2 | Limpide |
| GLU-1 | Turbide |
| GLU-2 | Turbide |
| GLU-3 | Turbide |
| LIS-1 | Limpide |
| LIS-2 | Limpide |

Parmi les insulines évaluées, seules l'insuline humaine, l'insuline lispro et l'insuline humaine A21G permettent d'obtenir une formulation homogène et limpide avec le pramlintide à pH 4, attestant de la solubilité des espèces. Les insulines aspart et glulisine ne conviennent pas pour obtenir une formulation limpide avec le pramlintide à pH 4,0.

### Exemple 4. Etude du temps de latence de fibrillation.

### Principe

La mauvaise stabilité d'un peptide peut conduire à la formation de fibrilles amyloïdes, définies comme des structures macromoléculaires ordonnées. Celles-ci peuvent éventuellement conduire à la formation de gel au sein de l'échantillon.

L'essai de suivi de la fluorescence de la thioflavine T (ThT) est utilisé pour analyser la stabilité physique des formulations. La thioflavine T est une petite molécule sonde ayant une signature de fluorescence caractéristique lorsqu'elle se lie à des fibrilles de type amyloïdes (Naiki et al. (1989) Anal. BioChem. 177, 244-249 ; LeVine (1999) Methods. Enzymol. 309, 274-284).

Cette méthode permet de suivre la formation de fibrilles pour de faibles concentrations de ThT au sein de formulations non diluées. Ce suivi est réalisé dans des conditions de stabilité accélérées : sous agitation et à 37°C.

### Conditions expérimentales

Les échantillons ont été préparés juste avant le début de la mesure. La préparation de chaque composition est décrite dans l'exemple associé. La thioflavine T a été ajoutée dans la composition à partir d'une solution mère concentrée de manière à induire une dilution négligeable de la composition. La concentration de thioflavine T dans la composition est 40 µM. Un volume de 150 µL de la composition a été introduit au sein d'un puit d'une plaque 96 puits. Chaque composition a été analysée en triplicat au sein d'une même plaque. La plaque a été scellée par du film transparent afin d'éviter l'évaporation de la composition.

Cette plaque a ensuite été placée dans l'enceinte d'un lecteur de plaques (EnVision 2104 Multilabel, Perkin Elmer). La température est réglée à 37°C, et une agitation latérale de 960 rpm avec 1 mm d'amplitude est imposée.

Une lecture de l'intensité de fluorescence dans chaque puit est réalisée avec une longueur d'onde d'excitation de 442 nm, et une longueur d'onde d'émission de 482 nm au cours du temps.

Le processus de fibrillation se manifeste par une forte augmentation de la fluorescence après un délai appelé temps de latence.

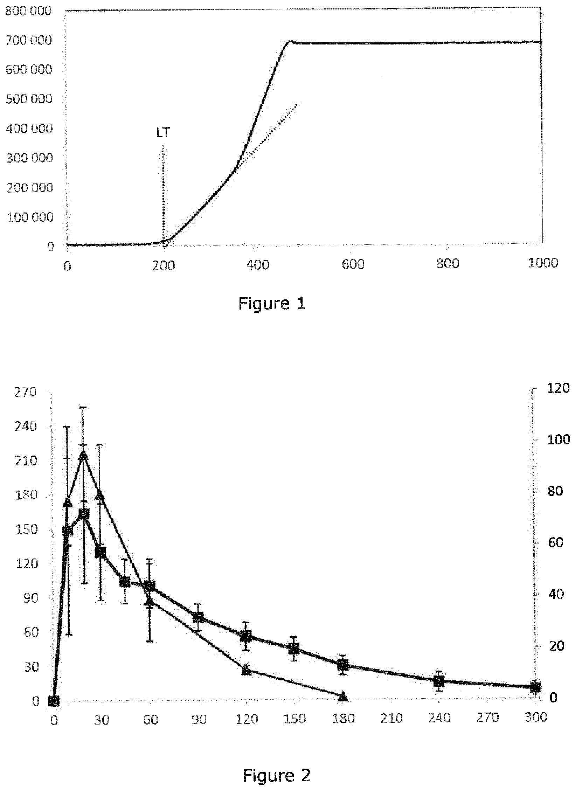

Pour chaque puit, ce délai a été déterminé graphiquement comme l'intersection entre la ligne de base du signal de fluorescence et la pente de la courbe de fluorescence en fonction du temps déterminée pendant la forte augmentation initiale de fluorescence, tel que montré en Figure 1. La valeur de temps de latence reportée correspond à la moyenne des temps de latence des 3 puits.

Les solutions de pramlintide et d'insuline limpides à pH 3,5 et 4,0 de l'exemple précédent sont ensuite soumises au test de fibrillation en présence de ThT.

Le temps de latence renseigné dans le Tableau 3 correspond à la moyenne des 3 mesures, l'intervalle d'incertitude correspond à l'écart moyen entre ces 3 résultats.

**Tableau 3 : Temps de latence des solutions d'insuline et de pramlintide.**

| **Composition** | **Temps de latence (h)** |
|---|---|
| A21-1 | 13,7 +/- 0,8 |
| A21-2 | 10,4 +/- 1,8 |
| A21-3 | 15,6 +/- 5,3 |
| RHI-1 | 5,0 +/- 0,7 |
| RHI-2 | 1,7 +/- 0 |
| ASP-2 | 2,0 +/- 0 |
| LIS-1 | 1,8 +/- 0,1 |
| LIS-2 | 4,3 +/-0,4 |

De façon inattendue, les formulations contenant l'insuline humaine A21G ont des temps de latence de fibrillation bien supérieurs à ceux des insulines commerciales testées à pH 3,5 ou à pH 4,0, en particulier aux insulines analogues rapides, lispro et aspart.

### Exemple 5. Etude du temps de latence de fibrillation en présence de Tween 20

Dans le Tableau 4 sont présentés les temps de latence de solutions d'insuline humaine A21G et de pramlintide à pH 4 en présence de Tween 20.

**Tableau 4 : Temps de latence des solutions d'insuline humaine A21G et de pramlintide en présence de Tween 20.**

| **Composition** | **Temps de latence (h)** |
|---|---|
| A21-4 | 38,1 +/-8,8 |
| A21-5 | 48,3 +/-9,2 |

La stabilité physique est donc améliorée en présence de Tween 20 à 10 µg/mL

### Exemple 6. Stabilité physique des formulations à 30°C en condition statique

Des cartouches en verre remplies avec 1 mL de composition sont placées dans une étuve maintenue à 30°C. Ces cartouches sont inspectées visuellement afin de détecter l'apparition de particules visibles ou d'une turbidité. Cette inspection est réalisée selon les recommandations de la Pharmacopée Européenne (EP 2.9.20) : les cartouches sont soumises à un éclairage d'au moins 2000 Lux et sont observées face à un fond blanc et un fond noir. Ces résultats sont en accord avec la pharmacopée US (USP <790>).

**Tableau 5 : Stabilité physique des solutions d'insuline et de Pramlintide à 30°C en condition statique.**

| **Composition** | **Aspect visuel après 4 semaines** |
|---|---|
| A21-4 | Limpide |
| A21-5 | Limpide |
| A21-2 | Limpide |
| A21-1 | Limpide |
| A21-3 | Limpide |
| ASP-2 | Trouble |

L'insuline aspart formulée avec le pramlintide à pH 3,5 est moins stable que l'insuline humaine A21G formulée avec pramlintide à pH 3,5 ou 4,0.

### Exemple 7. Etude de stabilité physique de l'insuline humaine A21G avec l'exénatide et le lixisénatide.

Les formulations A21-6 et A21-7 sont mises en cartouches puis placées à 30°C pendant 4 semaines. Les temps de latence de fibrillation sont mesurés pour des formulations fraichement préparées et sont présentées dans le tableau 6.

**Tableau 6 : Temps de latence et stabilité physique à 30°C en condition statique des solutions d'insuline humaine A21G et d'exénatide ou de lixisénatide.**

| **Composition** | **Temps de latence (h)** | **Aspect visuel après 4 semaines à 30 °C** |
|---|---|---|
| A21-6 | 18,1 +/- 5,6 | Limpide |
| A21-7 | 33,0 +/- 9,7 | Limpide |

### Exemple 8. Stabilité chimique d'une formulation d'insuline humaine A21G et de pramlintide

Toutes les formulations sont à pH 4,0 ou à pH 3,5 et contiennent 100 U/mL d'insuline humaine A21G, 1 mg/mL de pramlintide, 25 mM de m-crésol et 184 mM de glycérine. Les formulations sont stockées dans des cartouches en verre et placées à 30°C en condition statique. L'insuline humaine A21G et le pramlintide sont dosés par chromatographie liquide (HPLC) en phase inverse. Les mesures sont présentées dans le tableau 7.

**Tableau 7 : Evolution des concentrations d'insuline (a) en U/mL et de pramlintide (b) en mg/mL**

| **Composition** | **Concentration au temps initial** | | **Concentration après 4 semaines** | |
|---|---|---|---|---|
| | (a) | (b) | (a) | (b) |
| A21-1 | 105 | 1,05 | 103 | 1,02 |
| A21-2 | 105 | 1,02 | 106 | 1,03 |
| A21-3 | 101 | 1,12 | 105 | 1,03 |
| RHI-1 | 101 | 1,6 | 76 | 1,03 |
| RHI-2 | 99 | 1,02 | 62 | 1,03 |
| LIS-1 | 105 | 1,07 | 89 | 1,03 |
| LIS-2 | 106 | 1,05 | 82 | 1,04 |
| ASP-2 | 104 | 1,05 | 55 | 1,02 |

Les formulations contenant l'insuline humaine A21G et le pramlintide présentent une bonne stabilité chimique après 4 semaines à 30°C. Les formulations de pramlintide avec les insulines commerciales se dégradent rapidement à pH 4,0, et encore plus vite à pH 3,5.

### Exemple 9. Etudes de pharmacocinétique et pharmacodynamie chez le chien

Etude de pharmacocinétique et pharmacodynamie chez le chien de la composition insuline humaine A21G (100 U/mL, c'est-à-dire 3,5 mg/ml) et pramlintide (0,6 mg/mL). La formulation testée est à pH 4,0 et contient 25 mM de m-crésol et 184 mM de glycérine (Formulation A21-8).

Quatre animaux mis à jeun depuis 18 heures environ ont été injectés par voie sous-cutanée dans le cou à la dose de 0,2 U/kg d'insuline et 0,12 µg/kg de pramlintide. Dans l'heure précédant l'injection, un ou plusieurs prélèvements sanguins sont réalisés afin de déterminer le niveau basal de glucose, d'insuline et de pramlintide. Des prélèvements sanguins sont ensuite réalisés pendant 5 heures après administration de la formulation. La glycémie est déterminée au moyen d'un glucomètre. Les niveaux d'insuline et de pramlintide dans le plasma sont déterminés par un test ELISA.

Les paramètres pharmacocinétiques de la formulation A21-8 sont estimés à partir des concentrations plasmatiques d'insuline et de pramlintide corrigées des valeurs basales. Une analyse non compartimentale standard est réalisée à l'aide du logiciel Phoenix WinNonlin (version 7, Certara). Les valeurs des paramètres (moyenne ± écart-type) sont reportées dans les tableaux 8 et 9 ci-dessous :

**Tableau 8 : paramètres de PK de l'analogue de l'insuline totale**

| **Formulation** | **Tmax insuline (min)** | **Cmax insuline (pmol/L)** | **AUCO-last insuline (min^{∗}pmol/L)** |
|---|---|---|---|
| A21-8 | 38 ± 26 | 218 ± 141 | 16165 ± 4160 |

**Tableau 9 : paramètres de PK du pramlintide**

| **Formulation** | **Tmax pramlintide (min)** | **Cmax pramlintide (pmol/L)** | **AUCO-last pramlintide (min^{∗}pmol/L)** |
|---|---|---|---|
| A21-8 | 20 ± 8 | 104 ± 39 | 5121 ± 2961 |

Les profils pharmacocinétiques (PK) moyens de l'insuline totale (carrés) et du pramlintide (triangles) dans le plasma sont présentés dans la Figure 2. Les profils de glycémies moyennes exprimées en pourcentages du niveau basal sont représentés dans la Figure 3.

Il est constaté que le pramlintide et l'insuline humaine A21G ont tous les deux des cinétiques d'absorption prandiales donnant lieu à une activité hypoglycémiante précoce suivie d'un retour à un niveau proche de la glycémie basale après 5 heures post-administration. Ces résultats de pharmacocinétique et de pharmacodynamie indiquent clairement que la Formulation A21-8 est compatible avec une utilisation lors des repas.

### Exemple 10 : Etudes de pharmacocinétique du pramlintide chez le cochon

Etude de pharmacocinétique chez le cochon de la composition insuline humaine A21G (3,5 mg/mL équivalent à 100 U/mL d'insuline) et pramlintide (0,6 mg/mL).

Des cochons domestiques d'environ 50 kg, préalablement cathétérisés au niveau de la jugulaire, sont mis à jeun 2,5 heures avant le début de l'expérience. Dans l'heure précédant l'injection d'insuline, 3 prélèvements sanguins sont réalisés afin de déterminer le niveau basal de glucose et d'insuline.

L'injection des formulations d'insuline humaine A21G combinée à du pramlintide (A21-9) ou de pramlintide (PRAM) à la dose de 0,2 U d'insuline/kg et 1,2 µg de pramlintide/kg est réalisée en sous-cutané au niveau du flanc de l'animal à l'aide d'un stylo à insuline (Novo, Sanofi ou Eli Lilly) équipé d'une aiguille 31 G.

Afin de déterminer les concentrations plasmatiques en pramlintide, des prélèvements sanguins sont réalisés aux temps suivants : 4, 8, 12, 16, 20, 30, 40, 50, 60, 70, 80, 100, 120, 150 et 180 minutes. Après chaque prélèvement, le cathéter est rincé avec une solution diluée d'héparine.

### Résultats de pharmacocinétique de la solution d'insuline humaine A21G et de pramlintide A21-9 et de la solution de pramlintide PRAM chez le cochon

Les résultats de 3 études réalisées sur une même cohorte de cochons ont été mis en commun pour comparer les pharmacocinétiques du pramlintide entre la formulation A21-9 et la formulation PRAM. Les paramètres pharmacocinétiques des formulations A21-9 et PRAM sont estimés à partir des concentrations plasmatiques de pramlintide corrigées des valeurs basales. Une analyse non compartimentale standard est réalisée à l'aide du logiciel Phoenix WinNonlin (version 7, Certara). Les valeurs des paramètres (moyenne ± écart-type) sont reportées dans le tableau ci-après.

**Tableau 10 : paramètres de PK du pramlintide des compositions A21-9 et PRAM**

| Formulation | Peptides (mg/mL) | N | tₘₐₓ pramlintide (min) | AUC₀₋₃₀ₘᵢₙ pramlintide (min^{∗}pmol/L) | AUC₀₋ₜ pramlintide (min^{∗}pmol/L) |
|---|---|---|---|---|---|
| A21-9 | insuline humaine A21G (3,5) pramlintide (0,6) | 34 | 42,1 ± 22,4 | 2461 ± 1467 | 13642 ± 5934 |
| PRAM | Pramlintide (0,6) | 34 | 23,2 ± 15,0 | 4357 ± 3127 | 14806 ± 7872 |
| Valeur p (significatif si p<0.05) | Comparaison A21-9 versus PRAM | 34 | 0,0004 | 0,0007 | 0,8018 |

Avec tmax= temps nécessaire pour observer la concentration plasmatique maximale ; AUC₀₋₃₀ₘᵢₙ= aire sous la courbe des concentrations plasmatiques en fonction du temps entre 0 et 30 min après injection ; AUC₀₋ₜ= aire sous la courbe des concentrations plasmatique en fonction du temps entre 0 et la dernière concentration quantifiable après injection

Les résultats de pharmacocinétique du pramlintide obtenus avec les formulations A21-9 et PRAM sont présentés sur la Figure 4. L'analyse de ces profils et des paramètres indique que la combinaison de l'insuline humaine A21G et du pramlintide (formulation A21-9, courbe tracée avec les carrés) conduit à un ralentissement significatif de l'absorption du pramlintide par rapport au pramlintide seul (formulation PRAM, courbe tracée avec les triangles). La formulation A21-9 conduit à un pic plasmatique (tₘₐₓ) significativement retardé (environ 18 min, p<0,05) et à une exposition plasmatique au pramlintide précoce (AUC₀₋₃₀ₘᵢₙ) significativement diminuée (environ 43%, p<0,05) par rapport à la formulation PRAM. D'autre part, l'exposition plasmatique au pramlintide totale (AUC₀₋ₜ) apparait similaire entre les deux formulations suggérant des biodisponibilités comparables.

### Exemple 11. Etude de la consommation alimentaire chez le rat après injection de compositions contrôle et de compositions comprenant de l'insuline humaine A21G et/ou du pramlintide

Cette étude a été effectuée sur une population de 40 rats mâles Sprague Dawley d'au moins 6 semaines.

Les rats ont eu un accès libre à la nourriture et à l'eau, hormis une période de 6 heures de jeûne précédant l'injection sous-cutanée des compositions décrites dans le tableau ci-dessous.

**Tableau 11 : compositions injectées aux rats et nombre de rats traités**

| Composition | Contrôle | Humulin^{®} | PRAM | A21-9 |
|---|---|---|---|---|
| [Pramlintide] (mg/ml) | - | - | 1 | 0,6 |
| Dose de Pramlintide (µg/kg) | - | - | 60 | 60 |
| Type d'insuline [Insuline] (U/ml) | - | Humaine 100 | - | Humaine A21G 100 |
| Dose d'insuline (U/kg) | - | 10 | - | 10 |
| Nombre de rats | 10 | 10 | 10 | 10 |

La composition contrôle est une solution saline, c'est-à-dire une solution aqueuse comprenant 150 mM de NaCl.

La composition Humulin^{®} R est une solution commerciale d'insuline humaine commercialisée par ELI LILLY. Ce produit est une insuline humaine à 100 U/ml. Les excipients d'Humulin^{®} R sont le glycérol, le méta-crésol, l'hydroxyde de sodium et l'acide chlorhydrique pour l'ajustement du pH (pH 7,0-7,8) et de l'eau.

A t0, immédiatement après l'injection, la nourriture est distribuée (environ 100 g par rat). La prise alimentaire (moyenne cumulée) est mesurée une, deux, et trois heures après t0, soit t+1h, t+2h et t+3h.

Les résultats sont présentés dans le tableau suivant :

**Tableau 12 : prise alimentaire 1, 2 et 3 heures après injection**

| Compositions | Contrôle | Humulin | PRAM | A21-9 |
|---|---|---|---|---|
| Prise alimentaire à t+1h (q) | 3,8 | 4.7 | 1,5 | 3,2 |
| Prise alimentaire à t+2h (g) | 4,4 | 5,3 | 3,3 | 4,4 |
| Prise alimentaire à t+3h (g) | 5,9 | 6,9 | 4,2 | 5,3 |

Ces résultats montrent que la composition A21-9, combinant l'insuline A21G et le pramlintide permet non seulement de diminuer la prise alimentaire induite par l'injection d'insuline, mais elle permet également de limiter la prise alimentaire à un niveau inférieur ou égal à celui du groupe témoin ayant subit une injection de composition Contrôle (solution saline).

## Revendications

1. Composition sous forme d'une solution aqueuse injectable, dont le pH est compris de 3,5 à 4,4 comprenant au moins l'insuline humaine A21G dite regular et au moins un suppresseur de glucagon à action prandiale.

2. Composition selon la revendication 1, **caractérisée en ce que** le suppresseur de glucagon à action prandiale est choisi dans le groupe constitué d'un analogue de l'amyline ou un agoniste du récepteur de l'amyline ou un analogue du GLP-1 ou un agoniste du récepteur au GLP-1 (GLP-1 RA).

3. Composition selon l'une quelconque des revendications précédentes, **caractérisée en ce que** le suppresseur de glucagon à action prandiale est un analogue de l'amyline ou un agoniste du récepteur de l'amyline.

4. Composition selon l'une quelconque des revendications précédentes, **caractérisée en ce que** le peptide suppresseur de glucagon à action prandiale est le pramlintide.

5. Composition selon l'une quelconque des revendications précédentes, **caractérisée en ce que** le peptide suppresseur de glucagon à action prandiale est l'exenatide.

6. Composition selon l'une quelconque des revendications précédentes, **caractérisée en ce que** le peptide suppresseur de glucagon à action prandiale est le lixisenatide.

7. Composition selon la revendication 1, **caractérisée en ce que** la concentration en insuline humaine A21G est comprise de 2 à 20 mg/mL.

8. Composition selon la revendication 1, **caractérisée en ce que** la concentration en insuline humaine A21G est de 3,5 mg/mL.

9. Composition selon l'une quelconque des revendications précédentes, **caractérisée en ce que** la concentration en peptide suppresseur de glucagon à action prandiale est comprise de 0,01 à 10 mg/mL.

10. Composition selon l'une quelconque des revendications précédentes, **caractérisée en ce que** le pH de la solution est comprise de 3,8 à 4,2.

11. Composition selon l'une quelconque des revendications précédentes, **caractérisée en ce que** le pH de la solution est de 4,0.

12. Composition selon l'une quelconque des revendications précédentes, destinée à être utilisée dans une méthode de traitement du diabète **caractérisée en ce qu'**elle est administrée en bolus avant les repas.

13. Composition selon l'une quelconque des revendications 1 à 11, destinée à être utilisée dans une méthode de traitement du diabète **caractérisée en ce qu'**elle est administrée pour améliorer le contrôle de la glycémie postprandiale.

14. Composition selon l'une quelconque des revendications 1 à 11, destinée à être utilisée dans une méthode de traitement du diabète **caractérisée en ce qu'**elle est administrée pour améliorer le contrôle de la glycémie postprandiale et pour diminuer les effets indésirables du pramlintide.

15. Composition selon l'une quelconque des revendications 1 à 11, destinée à être utilisée dans une méthode de traitement du diabète **caractérisée en ce qu'**elle permet de diminuer la prise alimentaire induite par l'insuline.

## Patentansprüche

1. Zusammensetzung in Form einer injizierbaren wässrigen Lösung, deren pH-Wert zwischen 3,5 und 4,4 liegt, umfassend mindestens das sogenannte Humaninsulin A21G und mindestens einen Suppressor von postprandialem Glucagon.

2. Zusammensetzung nach Anspruch 1, **dadurch gekennzeichnet, dass** der Suppressor von postprandialem Glucagon ausgewählt ist aus der Gruppe, bestehend aus einem Analogon von Amylin oder einem Amylin-Rezeptoragonisten oder einem Analogon von GLP-1 oder einem GLP-1-Rezeptoragonisten (GLP-1-RA).

3. Zusammensetzung nach einem der vorhergehenden Ansprüche, **dadurch gekennzeichnet, dass** der Suppressor von postprandialem Glucagon ein Analogon von Amylin oder ein Amylin-Rezeptoragonist ist.

4. Zusammensetzung nach einem der vorhergehenden Ansprüche, **dadurch gekennzeichnet, dass** es sich bei dem Suppressorpeptid von postprandialem Glucagon um Pramlintid handelt.

5. Zusammensetzung nach einem der vorhergehenden Ansprüche, **dadurch gekennzeichnet, dass** es sich bei dem Suppressorpeptid von postprandialem Glucagon um Exenatid handelt.

6. Zusammensetzung nach einem der vorhergehenden Ansprüche, **dadurch gekennzeichnet, dass** es sich bei dem Suppressorpeptid von postprandialem Glucagon um Lixisenatid handelt.

7. Zusammensetzung nach Anspruch 1, **dadurch gekennzeichnet, dass** die Konzentration an Humaninsulin A21G zwischen 2 und 20 mg/ml liegt.

8. Zusammensetzung nach Anspruch 1, **dadurch gekennzeichnet, dass** die Konzentration an Humaninsulin A21G 3,5 mg/ml beträgt.

9. Zusammensetzung nach einem der vorhergehenden Ansprüche, **dadurch gekennzeichnet, dass** die Konzentration des Suppressorpeptids von postprandialem Glucagon zwischen 0,01 und 10 mg/ml liegt.

10. Zusammensetzung nach einem der vorhergehenden Ansprüche, **dadurch gekennzeichnet, dass** der pH-Wert der Lösung zwischen 3,8 und 4,2 liegt.

11. Zusammensetzung nach einem der vorhergehenden Ansprüche, **dadurch gekennzeichnet, dass** der pH-Wert der Lösung 4,0 beträgt.

12. Zusammensetzung nach einem der vorhergehenden Ansprüche, die dazu vorgesehen ist, bei einem Verfahren zur Behandlung von Diabetes genutzt zu werden, **dadurch gekennzeichnet, dass** sie als Bolus vor den Mahlzeiten verabreicht wird.

13. Zusammensetzung nach einem der Ansprüche 1 bis 11, die dazu vorgesehen ist, bei einem Verfahren zur Behandlung von Diabetes genutzt zu werden, **dadurch gekennzeichnet, dass** sie verabreicht wird, um die Kontrolle des postprandialen Blutzuckerspiegels zu verbessern.

14. Zusammensetzung nach einem der Ansprüche 1 bis 11, die dazu vorgesehen ist, bei einem Verfahren zur Behandlung von Diabetes genutzt zu werden, **dadurch gekennzeichnet, dass** sie verabreicht wird, um die Kontrolle des postprandialen Blutzuckerspiegels zu verbessern und um die unerwünschten Nebenwirkungen von Pramlintid zu verringern.

15. Zusammensetzung nach einem der Ansprüche 1 bis 11, die dazu vorgesehen ist, bei einem Verfahren zur Behandlung von Diabetes genutzt zu werden, **dadurch gekennzeichnet, dass** sie es ermöglicht, die insulininduzierte Nahrungsaufnahme zu verringern.

## Claims

1. A composition in the form of an injectable aqueous solution, the pH of which is from 3.5 to 4.4, comprising at least human insulin A21G referred to as regular and at least a glucagon suppressor with prandial action.

2. The composition according to claim 1, wherein the glucagon suppressor with prandial action is selected from the group consisting of an amylin analog or an amylin receptor agonist or a GLP-1 analog or a GLP-1 receptor agonist (GLP-1 RA).

3. The composition according to any one of the preceding claims, wherein the glucagon suppressor with prandial action is an amylin analog or an amylin receptor agonist.

4. The composition according to any one of the preceding claims, wherein the glucagon suppressor peptide with prandial action is pramlintide.

5. The composition according to any one of the preceding claims, wherein the glucagon suppressor peptide with prandial action is exenatide.

6. The composition according to any one of the preceding claims, wherein the glucagon suppressor peptide with prandial action is lixisenatide.

7. The composition according to claim 1, wherein the concentration of human insulin A21G is from 2 to 20 mg/mL.

8. The composition according to claim 1, wherein the concentration of human insulin A21G is 3.5 mg/mL.

9. The composition according to any one of the preceding claims, wherein the concentration of glucagon suppressor peptide with prandial action is from 0.01 to 10 mg/mL.

10. The composition according to any one of the preceding claims, wherein the pH of the solution is from 3.8 to 4.2.

11. The composition according to any one of the preceding claims, wherein the pH of the solution is 4.0.

12. The composition according to any one of the preceding claims, intended to be used in a diabetes treatment method, wherein it is administered in a bolus before meals.

13. The composition according to any one of claims 1 to 11, intended to be used in a diabetes treatment method, wherein it is administered to improve the control of postprandial glycemia.

14. The composition according to any one of claims 1 to 11, intended to be used in a diabetes treatment method, wherein it is administered to improve the control of postprandial glycemia and to decrease the adverse effects of pramlintide.

15. The composition according to any one of claims 1 to 11, intended to be used in a diabetes treatment method, wherein it enables to decrease the food consumption induced by insulin.
